# EUROPÄISCHE PATENTSCHRIFT

(11) **EP 3 372 278 B1**
(45) Veröffentlichungstag und Bekanntmachung des Hinweises auf die Patenterteilung: **27.01.2021**
(21) Anmeldenummer: 18159800.4
(22) Anmeldetag: 02.03.2018
(51) Int. Cl.: A61N 2/02, A61N 2/00

(54) **POSITIONIERHILFE FÜR TMS**
POSITIONING AID FOR TMS
AIDE AU POSITIONNEMENT POUR TMS

(30) Priorität: 06.03.2017 DE 102017104627
(43) Veröffentlichungstag der Anmeldung: 12.09.2018
(73) Patentinhaber: MAG & More GmbH, 81379 München (DE)
(72) Erfinder: WÖLFEL, Markus, 81377 München (DE); HIRSCHBECK, Christian, 81247 München (DE); HÄRINGER, Kerstin, 81371 München (DE)
(74) Vertreter: Grünecker Patent- und Rechtsanwälte PartG mbB

(56) Entgegenhaltungen:
- EP-A1- 2 878 336
- WO-A1-03/098268
- WO-A1-2005/075019
- WO-A1-2016/056326
- WO-A2-2008/130533
- DE-A1- 10 242 542
- DE-A1-102008 034 237

## Beschreibung

Die vorliegende Erfindung betrifft eine Vorrichtung zum Positionieren einer Magnetspule relativ zum Kopf eines Patienten sowie ein Verfahren zum Positionieren einer Magnetspule relativ zum Kopf eines Patienten.

Die transkranielle Magnetstimulation (TMS) ist ein nicht-invasives Verfahren zur Erregung oder Inhibition neuronaler Netzwerke im Gehirn. Neben der Gehirnforschung wird sie auch zur therapeutischen Behandlung z. B. von Depression eingesetzt.

Die korrekte und reproduzierbare Positionierung einer dabei verwendeten Magnetspule am Kopf des Patienten ist bei der TMS eine der wichtigsten Anforderungen. Dabei müssen für jeden Patienten individuelle Stimulationspunkte, sogenannte Hot-Spots, gesucht und bei jeder Sitzung wiedergefunden werden. Die Stimulationsspule muss am gewünschten Stimulationspunkt positioniert werden und muss während einer Sitzung durchgehend an dieser Position verbleiben. Zur Entlastung des Patientennackens während den Sitzungen wird entweder ein Vakuumkissen zur entspannten Lagerung des Kopfes oder ein Patientensessel mit integrierter Kopfauflage verwendet.

Im klinischen Alltag werden meist Kappen oder Badehauben für den Kopf verwendet, auf denen die individuellen Stimulationspunkte eines Patienten markiert werden. Zur Bestimmung eines geeigneten Stimulationsortes und einer geeigneten Stimulationsintensität wird der Kopf vermessen und mögliche Stimulationspunkte (sogenanntes 9-Punkte-Grid) werden eingezeichnet. Danach wird auf Basis des eingezeichneten Grids durch Abgabe von Stimulationspulsen in ausreichender Intensität und entsprechender Orientierung der Spule das Gehirnareal gesucht, welches die Handmotorik im Gehirn repräsentiert. Bei Stimulation des richtigen Ortes mit ausreichender Intensität wird die Reaktion des Daumens gemessen. Der Punkt mit der besten Reaktion wird auf der Haube markiert.Die individuelle Stimulationsstärke wird hier gemessen, und außerdem dient dieser Punkt als Ausgangspunkt für die Vermessung und Markierung des individuellen Therapiepunktes (z.B. über die 5 cm Regel). Dieser individuell vermessene Therapiepunkt wird auf der Kappe markiert und wird für jede Sitzung als Stimulationsort verwendet. Die Position der Spule muss während der Behandlung durch Halten der Spule oder durch Einspannen in eine Haltevorrichtung sichergestellt werden. Eine Fixierung bzw. Unterstützung des Kopfes wird hierbei nicht berücksichtigt und kann separat z.B. über ein Vakuumkissen oder eine entsprechende Nackenstütze am Patientensessel realisiert werden. Diese Methode stellt eine sehr einfach zu erlernende und kostengünstige Lösung dar. Allerdings können die Stimulationspunkte nie zu 100% genau wiedergefunden werden, da die Kappe nie ein zweites Mal exakt gleich aufgesetzt werden kann. Außerdem darf sich der Kopf des Patienten während der Stimulation nicht bewegen und die Spule muss so gehalten werden, dass sie die Position am Kopf für die Dauer der Stimulation nicht verändert.

Außerdem bekannt ist die Verwendung von Spulenpositionierungssystemen, bei denen die Spule auf Basis von Halte- und Messwerkzeugen individuell relativ zum Kopf positioniert wird. Bei diesen Systemen darf der Patient während der Stimulation die Lage des Kopfes nicht verändern, da die Spulenpositionierungssysteme der Bewegung des Kopfes nicht folgen können bzw. der Kontakt der Spule zum Kopf per Sensor gemessen wird und es zum Abbruch der Stimulation führt, wenn die Spule den Kontakt zum Kopf verliert (z.B. durch eine Kopfbewegung). Des Weiteren muss bei der Verwendung dieser Methode ein spezieller Patientensessel eigesetzt werden, der den Kopf des Patienten während der Stimulation stützt.

Zur hochpräzisen Positionierung der Spule können Neuronavigationssysteme eingesetzt werden. Diese verwenden MRT-Aufnahmen des jeweiligen Patienten um ein dreidimensionales Modell des Kopfes und des Gehirns zu erstellen. Mit Hilfe von Ultraschall- oder Infrarot-Trackingsystemen werden die Positionen von Spule und Kopf/Gehirn relativ zueinander in Echtzeit bestimmt und im 3D-Modell dargestellt. Hiermit kann die Spule sehr einfach über dem zu stimulierende Areal positioniert und die Position der Spule abgespeichert werden. Obwohl das Finden des richtigen Stimulationsortes durch die Neuronavigation nicht unbedingt erleichtert wird (der Therapiepunkt muss wie bei der Verwendung der Kappen händisch vermessen werden), bietet diese Methodik durch diverse Speichermöglichkeiten der Spulen-Kopf-Koordinaten eine sehr gute Reproduzierbarkeit und Dokumentationsmöglichkeit des Stimulationsortes sobald dieser bestimmt ist. Allerdings müssen auch bei dieser Methode sowohl die von Hand oder über ein Haltesystem gehaltene Spule als auch der Kopf des Patienten über die gesamte Stimulationsdauer ruhig gehalten werden, da ansonsten die Stimulationsposition verlassen wird. Außerdem sind die Kosten für die Anschaffung eines solchen Systems sind sehr hoch. Des Weiteren muss der Anwender im Umgang mit Computer, Software und Navigationssystemen geschult sein.

Eine relativ neue Entwicklung ist ein TMS-Roboter als Art intelligenter Spulenhalter, der mit Hilfe eines Neuronavigationssystems die Spule automatisch an der in der Navigation markierten Position relativ zum Kopf des Patienten hält und sie bei einer Bewegung des Patienten nachführt. Der TMS-Roboter kann die Spule in drei oder mehr Richtungen bewegen und in Verbindung mit einem Sensor so die entsprechende Positionierung und den Kontakt der Spule zum Kopf sicherstellen. Nachteilig ist der große Platz-, Technologie- und Schulungsbedarf, um den teuren Roboter zu bedienen.

Aus der DE 102 42 542 A1 ist ein Positionierungssystem für die navigierte transkranielle Magnetstimulation bekannt. Das Positionierungssystem umfasst eine Stativstange als Halterung für einen Bügel, an welchem über zwei Drehaufhängungen um eine Schwenkachse schwenkbar ein zweiter Bügel befestigt ist. Auf dem zweiten Bügel ist eine Laufkatze motorisch bewegbar angeordnet. Die Stimulationsspule ist mittels einer motorischen Verstellmöglichkeit an der Laufkatze befestigt. Mittels einer motorischen Verschwenkung des zweiten Bügels um die Schwenkachse, eines motorischen Bewegens der Laufkatze entlang des zweiten Bügels und einer motorischen Verstellung der Verstellmöglichkeit an der Laufkatze lässt sich die Position der Stimulationsspule relativ zu dem Kopf eines Patienten einstellen. An der Stativstange ist zudem eine Auflage für den Kopf eines Patienten befestigt. Um den Kopf des Patienten ortsfest zu halten, weist die Auflage Fixierungselemente in Form eines Gurtes auf, welcher um die Stirn des Patienten geschnallt wird und dessen Kopf an die Auflage drückt und damit fixiert. Alternativ sollen Auflagen mit zusätzlichen form- und/oder kraftschlüssigen Klemmvorrichtungen oder Einspannungen für den Patientenkopf vorgesehen sein. Durch die Anbringung der Auflage als Fixierung des Patientenkopfes an der Stativstange sollen Patientenbewegungen reduziert werden. Die Stativstange kann beispielsweise an einem Sitz für den Patienten angebracht sein. Weder die Auflage für den Kopf noch die Fixierungselemente zum Fixieren des Kopfes an der Auflage sollen das Gesichtsfeld des Patienten einschränken. Die Behandlung kann dennoch für den Patienten unangenehm sein, da sein Kopf während des Einstellens der Position der Stimulationsspule sowie während der Behandlungsdauer nicht bewegt werden darf und kann. Dies kann insbesondere bei wiederholter Behandlung zu Verspannungen im Nackenbereich und/oder zu Kopfschmerzen führen.

Weitere Positionierungssysteme zur transkraniellen Magnetstimulation sind aus der US 6,926,660 B2, der US 2010/0113959 A1, der US 7,651,459 B2, der US 8,088,058 B2, der US 2015/0202453 A1, der EP 2 252 367 B1, der WO 2005/075019 A1 und der US 2016/0030762 A1 bekannt.

Aus der WO 2016/056326 A1 ist eine Vorrichtung zum Positionieren einer Magnetspule zur transkraniellen Magnetstimulation bekannt. Eine Kopfstütze für den Kopf des Patienten und eine Positioniereinheit sind jeweils an einem Trägerelement angebracht. Die Kopfstütze ist zum Anpassen ihrer Lage an die Lage des Kopfes des Patienten anpassbar. Unabhängig davon ist die Positioniereinheit zum korrekten Positionieren der Magnetspule einstellbar. Während der Behandlung soll der Patient die Lage seines Kopfes nicht verändern. Dies wird während der Behandlung permanent optisch überwacht. Wird detektiert, dass sich der Kopf nicht mehr in der eingestellten Lage befindet, wird eine Stromzufuhr zu der Magnetspule unterbunden. Zudem wird detektiert, wenn sich der Kopf des Patienten von der Kopfstütze entfernt. Auch in diesem Fall wird die Stromzufuhr zu der Magnetspule unterbunden.

Es ist Aufgabe der Erfindung, eine Vorrichtung zum Positionieren einer Magnetspule relativ zum Kopf eines Patienten sowie ein entsprechendes Positionierungsverfahren bereitzustellen, die bei dem Patienten auch bei längerer Haltedauer der Magnetspule möglichst kein oder wenig Unbehagen hervorrufen.

Diese Aufgabe wird bezüglich der Vorrichtung durch den Gegenstand von Anspruch 1, bezüglich des Verfahrens durch den Gegenstand von Anspruch 13 gelöst. Die abhängigen Ansprüche geben vorteilhafte Weiterbildungen der Erfindung an.

Erfindungsgemäß wird eine Vorrichtung zum Positionieren einer Magnetspule zur transkraniellen Magnetstimulation relativ zum Kopf eines Patienten bereitgestellt. Die Vorrichtung umfasst eine Kopfeinheit mit einer Stützauflage für den Kopf des Patienten und mit einer mit der Stützauflage verbundenen Positioniereinheit. Die Positioniereinheit umfasst eine Spulenaufnahme zum Halten der Magnetspule. Zudem umfasst die Vorrichtung einen raumfest installierbaren Haltearm, der die Kopfeinheit hält.

Der Haltearm kann beispielsweise an oder zumindest relativ zu einem Boden oder einer Wand eines Behandlungsraums oder einem Patientensessel raumfest befestigt werden. Es ist auch denkbar, den Haltearm an einem Gerätewagen anzubringen. "Raumfest installierbar" soll bezüglich des Haltearms heißen, dass seine Position und Ausrichtung nach der Installation zumindest soweit festgelegt ist, als dass er durch typische Bewegungen des Kopfes des Patienten, welche über die Stützauflage und die Kopfeinheit auf ihn einwirken, nicht bewegt oder verstellt wird. Vorzugsweise lässt sich der Haltearm wahlweise in einen Einstellmodus und in einen installierten Modus verbringen. In dem Einstellmodus kann der Haltearm zur geeigneten Positionierung in Bezug auf den Patienten mittels einer oder mehrerer Verstellmöglichkeiten verstellt werden. Beispielsweise ist eine Höhenverstellbarkeit durch teleskopisch ineinander gesteckte Teilelemente des Haltearms denkbar, um den Haltearm an eine Größe des Patienten anzupassen. Alternativ oder zusätzlich können mehrere Teilsegmente des Haltearms in dem Einstellmodus relativ zueinander verschwenkbar sein. Von dem Einstellmodus kann der Haltearm beispielsweise durch Arretieren der teleskopisch ineinander gesteckten Teilelemente im Bezug aufeinander und/oder durch Arretieren der Schwenkverbindungen seiner Teilsegmente in den installierten Modus verbracht werden, so dass der Haltearm raumfest installiert ist.

Erfindungsgemäß ist zwischen der Kopfeinheit und dem Haltearm eine bewegliche Verbindung vorgesehen. Die bewegliche Verbindung ist so ausgestaltet, dass eine Änderung einer durch den Kopf des Patienten auf die Stützauflage ausgeübten Kraft über die bewegliche Verbindung eine Relativbewegung zwischen der Kopfeinheit und dem Haltearm bewirkt. Die Verbindung zwischen der Kopfeinheit und dem Haltearm ist reversibel beweglich. Das heißt, dass eine von einer Änderung der durch den Kopf des Patienten auf die Stützauflage ausgeübten Kraft bewirkte Relativbewegung zwischen der Kopfeinheit und dem Haltearm rückgängig gemacht werden kann (im Gegensatz zu beispielsweise einer ratschenartigen Beweglichkeit). Es versteht sich, dass im Sinne der Erfindung die Relativbewegung zwischen der Kopfeinheit und dem Haltearm zerstörungsfrei erfolgen soll.

Die von dem Haltearm gehaltene Stützauflage übernimmt zumindest einen Teil des Gewichts des Kopfes und ermöglicht so eine komfortable und entspannte Lagerung des Kopfes auch über einen längeren Zeitraum hinweg. Da die Kopfeinheit von dem Haltearm gehalten wird, muss nicht das gesamte Gewicht der Kopfeinheit sowie einer daran befestigten Magnetspule von dem Kopf des Patienten getragen werden. Auch dies wirkt sich vorteilhaft auf eine entspannte Lagerung des Kopfes aus. Vorzugsweise ist das System selbsttragend.

Aufgrund der beweglichen Verbindung zwischen Kopfeinheit und Haltearm kann die Kopfeinheit auf eine Änderung der Kopfposition des Patienten oder auf eine Änderung einer Andrück- oder Auflagekraft des Kopfes auf die Stützauflage mit einer Relativbewegung in Bezug auf den Haltearm reagieren. Somit ist ein raumfestes Fixieren des Kopfes nicht mehr notwendig und der Patient hat jederzeit die Möglichkeit, selbst die Vorrichtung zu verlassen. Dies führt unter anderem dazu, dass der Patient die erfindungsgemäße Vorrichtung als besonders vertrauenserweckend aufnimmt. Zudem wird das Notfallprozedere bei einem epileptischen Anfall des Patienten oder Ähnlichem vereinfacht.

Vorzugsweise ist die bewegliche Verbindung zwischen der Kopfeinheit und dem Halteelement als permanent bewegliche Verbindung ausgebildet. Das kann heißen, dass die bewegliche Verbindung unverriegelbar ist und/oder keine Einrastposition aufweist. Hierdurch wird gewährleistet, dass sich die Position der Kopfeinheit unabhängig vom momentanen Betriebszustand an die von dem Kopf des Patienten auf die Stützauflage ausgeübte Kraft anpasst.

Die Positioniereinheit kann starr oder starr fixierbar mit der Stützauflage verbunden sein. So kann eine optimale Kraftübertragung zwischen der Stützauflage und dem Rest der Kopfeinheit erreicht werden. Insbesondere wird die von dem Kopf des Patienten auf die Stützauflage ausgeübte Kraft direkt an die Positioniereinheit mit der daran angebrachten Spule übertragen. Besonders bevorzugt ist es, wenn die Positioniereinheit in einem Einstellmodus gegenüber der Stützauflage verschiebbar ist und so an den Kopf des Patienten angepasst werden kann. Nach der Anpassung kann die Relativposition zwischen Positioniereinheit und Stützauflage mit einem Feststellelement festgelegt werden, so dass zwischen der Positioniereinheit und der Stützauflage eine starre Verbindung vorliegt.

Vorzugsweise ist die Stützauflage so ausgelegt, dass eine Änderung der durch den Kopf des Patienten auf die Stützauflage ausgeübten Kraft und/oder eine Bewegung des durch die Stützauflage gestützten Kopfes ein Mitführen der Kopfeinheit mit einer Bewegung des Kopfes bewirkt. Beispielsweise kann ein Drehen des Kopfes nach links oder rechts eine derartige Änderung der durch den Kopf auf die Stützauflage ausgeübten Kraft bewirken, dass sich die Kopfeinheit über die bewegliche Verbindung ebenfalls nach links oder rechts dreht, dem Kopf also folgt. Dabei kann die Relativposition zwischen der von der Spulenaufnahme gehaltenen Magnetspule und dem Kopf des Patienten zumindest im Wesentlichen konstant bleiben. Zumindest kann durch das Mitführen der Kopfeinheit eine Größe der Lageverschiebung zwischen Kopf und Magnetspule bei einer Bewegung des Kopfes abgeschwächt werden. Der Patient kann seinen Kopf also innerhalb eines gewissen Maßes bewegen, ohne dass die Position der Magnetspule neu eingestellt werden muss.

Vorzugsweise bleibt bei der Relativbewegung zwischen der Kopfeinheit und dem Haltearm über die bewegliche Verbindung die Lage der Spulenaufnahme relativ zu der Stützauflage gleich. So wird gewährleistet, dass die in der Spulenaufnahme gehaltene Magnetspule ihre Position relativ zu dem von der Stützauflage gestützten Kopf zumindest im Wesentlichen nicht ändert.

Die bewegliche Verbindung zwischen der Kopfeinheit und dem Haltearm kann gemäß einer bevorzugten Ausführungsform der durch den Kopf des Patienten auf die Stützauflage ausgeübten Kraft federnd entgegenwirken. Durch die federnde Gegenkraft erfährt der Kopf des Patienten eine optimale und an die Kopfposition und die jeweilige Auflagekraft des Kopfes angepasste Unterstützung. Wird durch den Kopf des Patienten eine erhöhte Kraft auf die Stützauflage ausgeübt, kann sich diese relativ zu dem Haltearm entgegen der Federwirkung bewegen. Hierdurch kann eine Rückstellkraft in die vorausgegangene Position entstehen, so dass bei einer erneuten Reduktion der durch den Kopf auf die Stützauflage ausgeübten Kraft die Kopfeinheit durch die Rückstellkraft in die vorangegangene Position bewegt wird. Zudem erfährt ein von dem Kopf des Patienten auf die Stützauflage ruckartig aufgebrachter Kraftimpuls eine Dämpfung, so dass keine ruckartigen Bewegungen der Kopfeinheit erfolgen.

Die bewegliche Verbindung zwischen der Kopfeinheit und dem Haltearm kann eine Translationskopplung umfassen. Die Translationskopplung kann eine lineare Translationsbewegung der Kopfeinheit in Bezug auf den Haltearm bezüglich einer Translationsrichtung erlauben, welche insbesondere zumindest im Wesentlichen parallel zur Sichtrichtung eines von der Stützauflage gestützten Kopfes liegt, also in einer Vorne-Hinten-Richtung des Kopfes. Der Patient kann seine Sitzposition also beispielsweise durch Nach-Vorne-oder-Hinten-Verschieben seines von der Stützauflage gestützten Kopfes anpassen.

Die Translationskopplung kann einen oder zwei den Bewegungsbereich der Translationskopplung begrenzende Anschläge haben. Ein erster Anschlag kann ein Lösen der Kopfeinheit von dem Haltearm verhindern. Ein zweiter Anschlag kann eine Bewegung der Stützauflage nach hinten begrenzen.

Die Translationskopplung kann eine Federeinrichtung umfassen. Die Federeinrichtung kann dazu ausgelegt sein, bei einer Vergrößerung einer zu der Translationsrichtung der Translationskopplung parallelen Komponente der durch den Kopf des Patienten auf die Stützauflage ausgeübten Kraft und einer damit einhergehenden linearen Translationsbewegung der Kopfeinheit entlang der Translationsrichtung gespannt zu werden. Bei einer nachfolgenden Verringerung der zu der Translationsrichtung parallelen Komponente der durch den Kopf auf die Stützauflage ausgeübten Kraft kann die Federeinrichtung die Kopfeinheit aufgrund der Spannung zu einer entgegengesetzten linearen Translationsbewegung beaufschlagen. Drückt der Patient seinen Kopf also beispielsweise gegen die Stützauflage nach hinten, kann sich die Stützauflage entgegen der Sichtrichtung des Kopfes nach hinten bewegen (Bewegung relativ zu dem Stützarm) und die Federeinheit wird gespannt. Verringert sich später durch eine Positionsänderung des Kopfes oder eine geänderte Körperspannung des Patienten die Andrückkraft nach hinten oder bewegt der Patient den Kopf nach vorne, wird die Stütztauflage aufgrund der Spannung der Federeinrichtung nach vorne nachgeführt. Bei der Federeinrichtung kann es sich beispielsweise um eine Spiralfeder oder eine pneumatische oder hydraulische Federeinrichtung handeln. Eine Federkonstante der Federeinrichtung kann bedarfsgemäß gewählt werden, um den Kopf des Patienten möglichst komfortabel zu stützen.

Zusätzlich oder alternativ zu der Translationskopplung kann die bewegliche Verbindung zwischen der Kopfeinheit und dem Haltearm eine oder mehrere Drehkopplungen umfassen, welche eine Schwenkbewegung der Kopfeinheit bezüglich des Haltearms erlauben. Vorzugsweise umfasst die bewegliche Verbindung eine erste Drehkopplung, die eine Schwenkbewegung der Kopfeinheit um eine erste Achse erlaubt, und/oder eine zweite Drehkopplung, die eine Schwenkbewegung der Kopfeinheit um eine zweite Achse erlaubt, und/oder eine dritte Drehkopplung, die eine Schwenkbewegung der Kopfeinheit um eine dritte Achse erlaubt. Durch das Vorsehen mehrerer, insbesondere dreier, Drehkopplungen kann die Kopfeinheit Bewegungen des von der Stützauflage gestützten Kopfes relativ frei folgen. Vorteilhafterweise sind die erste, die zweite und die dritte Achse zumindest im Wesentlichen jeweils rechtwinklig zueinander, so dass sich ein besonders großer Bewegungsspielraum der beweglichen Verbindung zwischen dem Haltearm und der Kopfeinheit ergibt.

Beispielweise kann die erste Achse bezüglich eines von der Stützauflage gestützten Kopfes zumindest im Wesentlichen parallel zu einer zur dorso-caudalen Achse (oben-unten Richtung) verlaufen. So kann die Kopfeinheit aufgrund der ersten Drehkopplung einer Bewegung des Kopfes folgen, welche einem Blick nach links oder rechts (wie bei einem Kopfschütteln) entspricht.

Die zweite Achse kann bezüglich eines von der Stützauflage gestützten Kopfes zumindest im Wesentlichen parallel zu einer Ohr-Ohr-Achse (laterale Richtung) verlaufen. So kann die Kopfeinheit aufgrund der zweiten Drehkopplung einer Bewegung des Kopfes folgen, welche einem Neigen nach oben oder unten (wie bei einem Nicken) entspricht.

Die dritte Achse kann bezüglich eines von der Stützauflage gestützten Kopfes zumindest im Wesentlichen parallel zu einer Dorso-rostral-Achse (Nase-Hinterkopf- oder Vorne-hinten-Richtung) verlaufen. So kann die Kopfeinheit aufgrund der dritten Drehkopplung einer Bewegung des Kopfes folgen, welche einem Kippen des Kopfes zu einer Schulter entspricht.

Auch bezüglich der Drehkopplung/en ist das Vorsehen jeweils eines oder mehrerer Endanschläge denkbar.

Vorzugsweise umfasst die Positioniereinheit zumindest ein Positionierelement, welches zum Einstellen der Lage der Spulenaufnahme relativ zu der Stützauflage verstellbar ist. Hierdurch lässt sich die von der Spulenaufnahme gehaltene Spule in eine geeignete Position mit Bezug auf den Kopf des Patienten bringen. Die Einstellung des Positionierelements ist vorzugsweise unabhängig von der Relativbewegung zwischen der Kopfeinheit und dem Haltearm über die bewegliche Verbindung. Insbesondere kann die Einstellung des Positionierelements verriegelbar sein.

Besonders vorteilhaft ist es, wenn die Positioniereinheit ein verschwenkbares erstes Positionierelement, insbesondere in Form eines Bügels, und ein verschiebbar daran angebrachtes zweites Positionierelement umfasst, an welchem die Spulenaufnahme fest oder verstellbar angebracht ist. Das erste Positionierelement und das zweite Positionierelement stellen zwei Freiheitsgrade der Einstellung der Spulenposition bereit.

Um eine Einstellung der Spulenposition für nachfolgende Sitzungen wiederverwenden zu können, kann zumindest eine Skala vorgesehen sein, an der eine Verschwenkstellung des ersten Positionierelements (z.B. Winkelmaßangabe) und/oder eine Verschiebeposition des zweiten Positionierelements (z.B. Längenmaßangabe) ablesbar sind. Die von der zumindest einen Skala abgelesenen Werte lassen für eine weitere Sitzung eine einfache und genaue Wiederherstellung der Spulenposition zu.

Vorzugsweise umfasst die Stützauflage eine Nackenstütze, welche insbesondere gepolstert sein kann.

Die Stützauflage kann zudem zu beiden Seiten der Nackenstütze jeweils eine Seitenstütze umfassen. Die Seitenstützen können jeweils derart schwenkbar, insbesondere mit Scharnieren, an der Nackenstütze angebracht sein, dass sie seitlich an einen von der Nackenstütze gestützten Kopf eines Patienten angelegt werden können und in einer Anlageposition gegenüber der Nackenstütze verriegelbar sind. Durch die Stützauflagen kann die Relativposition zwischen Kopf und Kopfeinheit besonders gut gehalten werden. Außerdem wird eine sehr gute Kraftübertragung zwischen Kopf und Stützauflage erreicht, so dass ein automatisches Nachführen der Kopfeinheit bei Bewegungen des Kopfes begünstigt wird.

Vorzugsweise lässt sich die Kopfeinheit zu Beginn der Einstellung gegenüber dem Kopf des Patienten in eine definierte Position bringen. Hierzu können beispielsweise Merkmale des Schädelknochens (z. B. Inion, Nasion und/oder Tragi) und/oder des Kopfes verwendet werden. Für zumindest zwei, vorzugsweise drei solcher Punkte kann ein Sucher an der Kopfeinheit vorgesehen sein. Wird die Kopfeinheit an zumindest zwei Merkmalen des Schädelknochens und/oder des Kopfes ausgerichtet, ist eine eindeutige Positionierung der Kopfeinheit bezüglich des Kopfes gegeben. So kann sichergestellt werden, dass die Kopfeinheit bei aufeinanderfolgenden Sitzungen bezüglich des Kopfes des Patienten identisch positioniert wird.

Sucher für Merkmale des Schädelknochens und/oder des Kopfes können beispielsweise als Öffnungen in der Positioniereinheit ausgebildet sein. Mittels einer oder mehrerer Öffnungen in der Positioniereinheit als Ohrpositionssucher lassen sich beispielsweise markante Ohrpositionen (z.B. Tragus) anvisieren. Zusätzlich oder alternativ können solche Ohrpositionssucher eine Quelle für einen Lichtstrahl, z.B. eine LED, umfassen, mit der die gesuchte Ohrposition anvisiert werden kann.

Alternativ oder zusätzlich kann die Stützauflage, insbesondere die Nackenstütze, eine Tastöffnung als Inionsucher zum Lokalisieren des Inions eines von der Stützauflage gestützten Kopfes umfassen. Die Tastöffnung bietet genügend Platz für einen Finger eines Anwenders und lässt sich mit dem Inion am Hinterkopf des Patienten ausrichten.

Die Erfindung betrifft zudem ein Verfahren zum Positionieren einer Magnetspule relativ zum Kopf eines Patienten. Die erfindungsgemäße Vorrichtung zum Positionieren einer Magnetspule relativ zum Kopf eines Patienten ist zum Durchführen des erfindungsgemäßen Verfahrens geeignet. Mit Bezug auf die Vorrichtung beschriebene Merkmale und Erläuterungen lassen sich auf das Verfahren übertragen und umgekehrt.

Ein erfindungsgemäßes Verfahren zum Positionieren einer Magnetspule relativ zum Kopf eines Patienten umfasst das Vorsehen eines raumfest (insbesondere relativ zu einem Behandlungsraum) installierten Haltearms. An dem Haltearm wird eine Kopfeinheit derart angebracht, dass der Haltearm die Kopfeinheit hält. Die Kopfeinheit umfasst eine Stützauflage für den Kopf des Patienten und eine mit der Stützauflage verbundene Positioniereinheit mit einer Spulenaufnahme zum Halten der Magnetspule. Die Kopfeinheit wird so an dem Haltearm angebracht, dass sie derart reversibel beweglich ist, dass eine Änderung einer durch den Kopf des Patienten auf die Stützauflage ausgeübten Kraft über die bewegliche Verbindung zwischen der Kopfeinheit und dem Haltearm eine Relativbewegung zwischen der Kopfeinheit und dem Haltearm bewirkt. Die Positioniereinheit wird zum Einstellen der Lage der Spulenaufnahme relativ zu der Stützauflage in eine gewünschte Behandlungsposition für die Magnetspule eingestellt.

Wie bereits mit Bezug auf die Vorrichtung beschrieben, unterstützt die an dem Haltearm angebrachte Stützauflage den Kopf des Patienten in seiner Position. Aufgrund der beweglichen Anbringung der Kopfeinheit an dem Haltearm ist ein Bewegen des durch die Stützauflage gestützten Kopfes des Patienten möglich, ohne die eingestellte Position der Magnetspule relativ zum Kopf zu verlieren. Ein raumfestes Fixieren des Kopfes des Patienten ist nicht mehr erforderlich.

Ersichtlicherweise wird durch die Erfindung der Patientenkomfort während des Positionierens der Magnetspule und während des Haltens derselben in einer eingestellten Position relativ zum Kopf deutlich verbessert. Darüber hinaus wird eine fehlerhafte Anwendung bzw. ein vorzeitiger Abbruch einer Anwendung durch Bewegung des Kopfes relativ zur Magnetspule weitgehend vermieden. Diese Verbesserung des Patientenkomforts und des Anwendungsdurchführung und die spezielle technische Realisierung des Positionierens und Haltens der Magnetspule relativ zum Kopf des Patienten, und nicht etwa eine Behandlung des Patienten mittels von der Magnetspule generierten elektromagnetischen Feldern, soll den Kern der Erfindung darstellen. Insbesondere können Therapieverfahren von der Erfindung ausgenommen sein.

Bei einer Änderung der durch den Kopf des Patienten auf die Stützauflage ausgeübten Kraft kann die Kopfeinheit aufgrund der beweglichen Verbindung zwischen der Kopfeinheit und dem Haltearm automatisch mit einer Bewegung des Kopfes des Patienten mitgeführt werden. Bei dem automatischen Mitführen der Kopfeinheit kann insbesondere die Einstellung der Positioniereinheit beibehalten werden und somit die Lage der Spulenaufnahme relativ zur Stützauflage gleich bleiben.

Das Verfahren kann, wie bereits in Bezug auf die Vorrichtung beschrieben, ein federndes Entgegenwirken durch die bewegliche Verbindung zwischen der Kopfeinheit und dem Haltearm gegen die durch den Kopf des Patienten auf die Stützauflage ausgeübte Kraft umfassen. Insbesondere kann die bewegliche Verbindung hierzu die oben beschriebene Translationskopplung aufweisen.

Im Folgenden wird die Erfindung anhand eines Ausführungsbeispiels mit Bezug auf die Figuren beschrieben. Dabei zeigen:
- Figur 1:: eine schematische ausschnittsweise Perspektivansicht einer Vorrichtung zum Positionieren einer Magnetspule relativ zum Kopf eines Patienten gemäß einer Ausführungsform, insbesondere zum Darstellen diverser Einstellmöglichkeiten der Kopfeinheit;
- Figur 2:: eine schematische Seitenansicht der Vorrichtung gemäß der Ausführungsform;
- Figur 3:: eine der Ansicht aus Fig. 1 entsprechende, schematische ausschnittsweise Perspektivansicht der Vorrichtung gemäß der Ausführungsform, insbesondere zur Darstellung der beweglichen Verbindung zwischen Haltearm und Kopfeinheit;
- Figur 4A:: eine schematische ausschnittsweise Rückansicht auf die Vorrichtung gemäß der Ausführungsform;
- Figur 4B:: eine schematische ausschnittsweise Seitenansicht auf die Vorrichtung gemäß der Ausführungsform;
- Figur 4C:: eine weitere schematische ausschnittsweise Rückansicht auf die Vorrichtung gemäß der Ausführungsform; und
- Figur 5:: eine schematische Detailansicht des Ausschnitts W aus Fig. 2.

Die Figuren zeigen eine Ausführungsform einer erfindungsgemäßen Vorrichtung 1 zum Positionieren einer Magnetspule 2 relativ zum Kopf eines Patienten. Die Magnetspule 2 kann zum Durchführen einer transkraniellen Magnetstimulation geeignet sein.

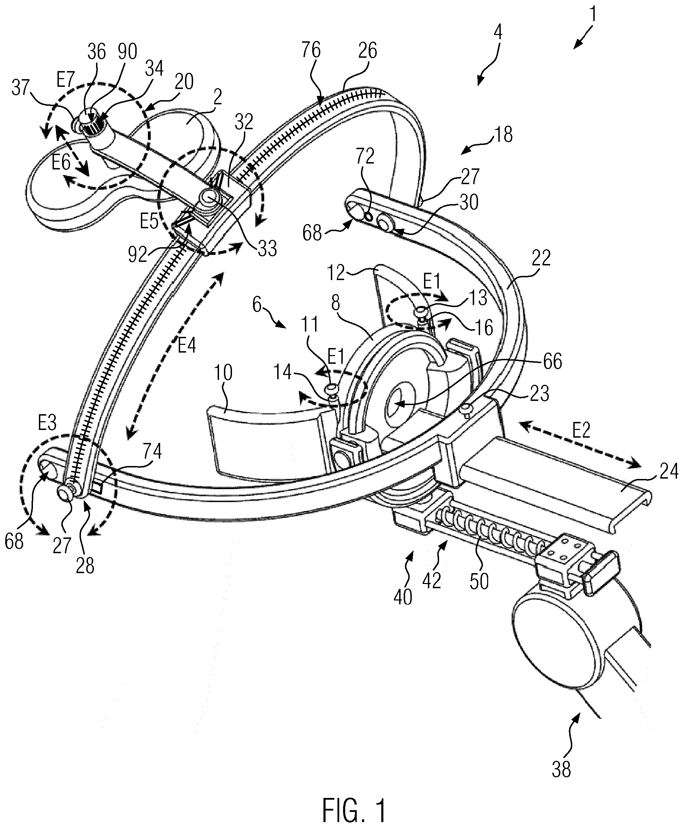

Wie zum Beispiel in dem in Figur 1 dargestellten Ausschnitt der Vorrichtung 1 ersichtlich, umfasst die Vorrichtung 1 eine Kopfeinheit 4 mit einer Stützauflage 6 für den Kopf des Patienten. Die Stützauflage 6 umfasst eine Nackenstütze 8 zum Anlehnen des Kopfes des Patienten. Zudem umfasst die Stützauflage 6 bezüglich einer Ohr-Ohr-Achse (laterale Richtung) eines von der Stützauflage 6 gestützten Kopfes zu beiden Seiten der Nackenstütze 8 jeweils eine Seitenstütze 10, 12. Die Seitenstützen 10, 12 sind jeweils über ein Scharnier 14, 16 schwenkbar an der Nackenstütze 8 angebracht. Die Verschwenkbarkeit der Seitenstützen 10, 12 ist in Fig. 1 jeweils durch die Pfeile E1 dargestellt. Nachdem der Patient sich mit seinem Hinterkopf gegen die Nackenstütze 8 gelehnt hat, können die Seitenstützen 10, 12 über die Scharniere 14, 16 seitlich an den Kopf des Patienten angelegt werden. In der angelegten Position können die Seitenstützen 10, 12 in Bezug auf die Nackenstütze 8 fixiert werden, beispielsweise mittels Schrauben 11, 13 oder anderen Feststellelementen.

Die Kopfeinheit 4 weist des Weiteren eine mit der Stützauflage 6 verbundene Positioniereinheit 18 mit einer Spulenaufnahme 20 zum Halten der Magnetspule 2 auf. Die Positioniereinheit 18 umfasst einen Basisbügel 22, mit welchem die Positioniereinheit 18 mit der Stützauflage 6 verbunden ist. Hierzu umfasst die Stützauflage 6 auf der dem Kopf des Patienten abgewandten Seite der Nackenstütze 8 eine sich nach hinten erstreckende Schiene 24. Auf dieser Schiene 24 ist der Basisbügel 22 der Positioniereinheit 18 parallel zu einer Vorne-Hinten-Richtung (Sichtrichtung) eines von der Stützauflage 6 gestützten Kopfes verschiebbar gelagert. Eine entsprechende Beweglichkeit ist durch den Pfeil E2 in Fig. 1 dargestellt. Durch eine Verschiebung des Basisbügels 22 entlang der Schiene 24 lässt sich die Position der Positioniereinheit 18 individuell an die Kopfgröße des Patienten anpassen, wie weiter unten detaillierter beschrieben wird. Ist eine gewünschte Einstellposition der Positioniereinheit 18 in Bezug auf die Stützauflage 6 erreicht, kann der Basisbügel 22 mittels einer Schraube 23 oder eines anderen geeigneten Feststellelements in seiner Position an der Schiene 24 festgelegt werden.

Zum Einstellen einer gewünschten Position der Magnetspule 2 in Bezug auf den von der Stützauflage 6 gestützten Kopf des Patienten umfasst die Positioniereinheit 18 des Weiteren ein erstes Positionierelement 26 in Form eines Bügels, welcher an seinen beiden Enden um eine zumindest im Wesentlichen parallel zu der Ohr-Ohr-Achse (laterale Richtung) stehende Achse schwenkbar an dem Basisbügel 22 angebracht ist. Eine entsprechende Schwenkbewegung ist in Fig. 1 durch den Pfeil E3 dargestellt. Die entsprechenden Schwenkverbindungen 28, 30 zwischen dem Basisbügel 22 und dem ersten Positionierelement 26 sind über das erste Positionierelement 26 synchronisiert. Nach dem Verbringen in eine geeignete Schwenkposition ist das erste Positionierelement 26 mittels eines geeigneten Feststellelements, wie beispielsweise einer Schraube 27, in seiner momentanen Schwenkposition bezüglich des Basisbügels 22 festlegbar. Es kann ausreichen, wenn eine der beiden Schwenkverbindungen 28, 30 festlegbar ist. Jedoch können auch für beide Schwenkverbindungen 28, 30 separate Feststellelemente vorgesehen sein.

Die Positioniereinheit 18 umfasst des Weiteren ein zweites Positionierelement 32, welches entlang des ersten Positionierelements 26 verschiebbar an dem ersten Positionierelement 26 gelagert ist. Die Verschiebbarkeit des zweiten Positionierelements ist in Fig. 1 durch den Pfeil E4 dargestellt. Das zweite Positionierelement 32 kann mittels eines geeigneten Feststellelements, beispielsweise einer Schraube 33, in seiner Position an dem ersten Positionierelement 26 festgelegt werden. Das zweite Positionierelement 32 trägt die Spulenaufnahme 20 für die Magnetspule 2.

In der gezeigten Ausführungsform ist die Spulenaufnahme 20 verstellbar an dem zweiten Positionierelement 32 angebracht. Insbesondere ist in der gezeigten Ausführungsform die Spulenaufnahme 20 um eine zumindest im Wesentlichen senkrecht zu der Verschieberichtung E4 des zweiten Positionierelements 32 stehende Achse gegenüber dem zweiten Positionierelement 32 drehbar (Drehrichtung E5). Diese Drehung der Spulenaufnahme 20 ist über das Feststellelement für die zweite Positioniereinheit 32, die Schraube 33, mit arretierbar. Es wäre auch denkbar, die Spulenaufnahme 20 fest an dem zweiten Positionierelement 32 anzubringen. In beiden Fällen lässt sich die Spulenaufnahme 20 in Bezug auf den von der Stützauflage 6 gestützten Kopf durch Verschwenken des ersten Positionierelements 26 gegenüber dem Basisbügel 22 (Pfeil E3) und/oder Verschieben des zweiten Positionierelements 32 entlang des ersten Positionierelements 26 (Pfeil E4) in eine gewünschte Position verbringen.

In der gezeigten Ausführungsform umfasst die Spulenaufnahme 20 eine Öffnung 34 zur Aufnahme eines an der Magnetspule 2 befestigter Bolzens 36. Mittels einer gleitenden Verschiebung des Bolzens 36 in der Öffnung 24 kann die Magnetspule 2 von oben auf die Kopfoberfläche zu oder von ihr weg bewegt werden, um den Abstand zwischen der Magnetspule 2 und dem Kopf zu optimieren (Pfeil E6). Zudem kann der Bolzen 36 um seine Erstreckungsachse herum gedreht werden, um gegebenenfalls auch eine Drehorientierung der Magnetspule 2 um eine im Wesentlichen senkrecht zur Kopfoberfläche stehende Achse einzustellen (Pfeil E7). Nach einem Heranführen der Magnetspule 2 an die Kopfoberfläche, ggf. bis zum Kontakt, und einer geeigneten Dreheinstellung um die Erstreckungsrichtung des Bolzens 36 herum kann die Magnetspule 2 in ihrer Position relativ zur Spulenaufnahme 20 durch Festlegen des Bolzens 36 in der Öffnung 34 der Spulenaufnahme 20 mittels eins geeigneten Feststellelements, wie z. B. einer Schraube 37, festgelegt werden.

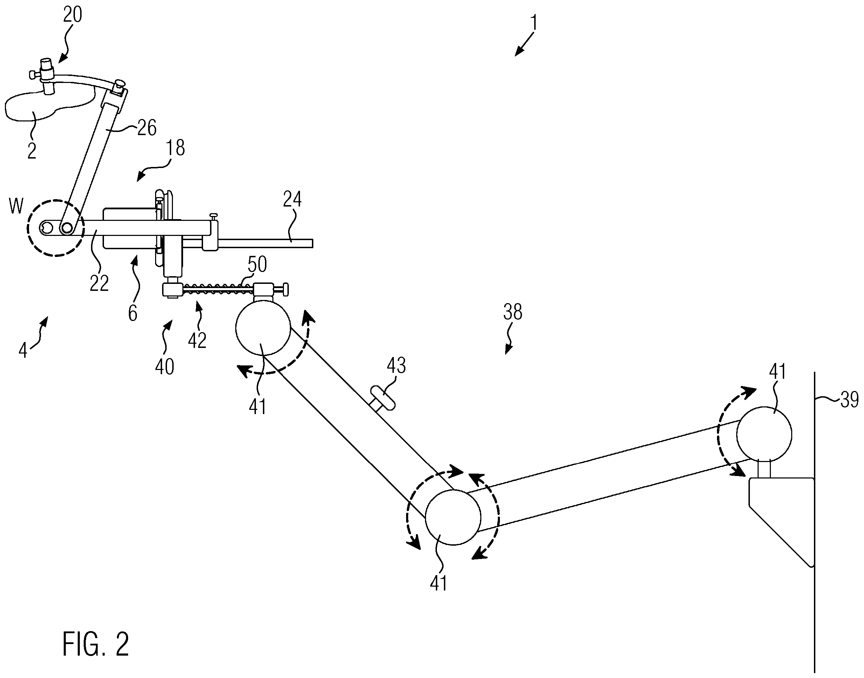

Die Vorrichtung 1 zum Positionieren der Magnetspule 2 relativ zum Kopf des Patienten umfasst des Weiteren einen die Kopfeinheit 4 haltenden, raumfest installierbaren Haltearm 38, welcher in Fig. 1 nur ausschnittsweise gezeigt ist. Die Seitenansicht nach Fig. 2 gibt einen guten Überblick über die Vorrichtung 1 gemäß der Ausführungsform und zeigt den vollständigen Haltearm 38. Wie dort gezeigt, ist der Haltearm 38 in der gezeigten Variante an einer Wand 39 raumfest angebracht. Es ist z.B. auch denkbar, den Haltearm 38 an einem Boden eines Behandlungsraums, an einem Gerätewagen oder an einem Patientensessel oder zumindest relativ dazu raumfest anzubringen. "Raumfest installierbar" soll bezüglich des Haltearms 38 heißen, dass seine Position und Ausrichtung nach der Installation zumindest soweit festgelegt ist, als dass er durch typische Bewegungen des Kopfes des Patienten, welche über die Stützauflage 6 und die Kopfeinheit 4 auf ihn einwirken, nicht bewegt oder verstellt wird.

Der dargestellte Haltearm 38 lässt sich wahlweise in einen Einstellmodus und in einen installierten Modus verbringen. In dem Einstellmodus kann der Haltearm 38 zur geeigneten Positionierung in Bezug auf den Patienten mittels zwischen Teilsegmenten des Haltearms 38 vorgesehenen gelenkigen Verstellmöglichkeiten 41 verstellt werden. Von dem Einstellmodus kann der Haltearm 38 durch Arretieren der gelenkigen Verstellmöglichkeiten 41 mittels eines gemeinsamen Betätigungselements 43 in den installierten Modus verbracht werden, so dass der Haltearm 38 raumfest installiert ist.

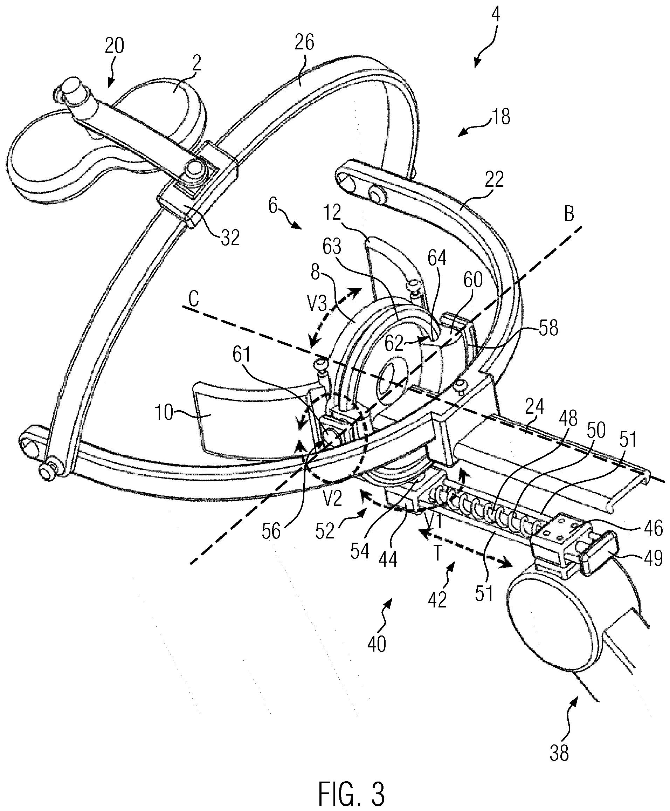

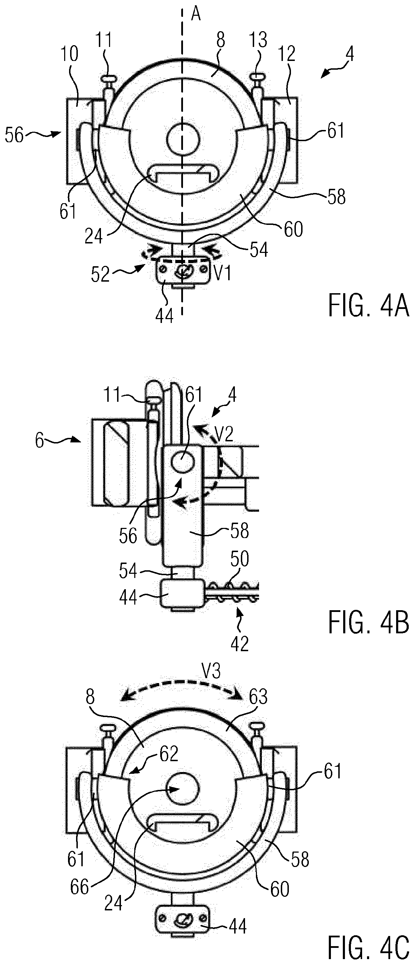

Zwischen der Kopfeinheit 4 und dem Haltearm 38 ist eine reversibel bewegliche Verbindung 40 vorgesehen. Diese ist in den Fig. 1 und 2 zumindest teilweise erkennbar. Der Übersichtlichkeit halber wird für die Beschreibung der beweglichen Verbindung 40 aber vornehmlich auf die Fig. 3 und 4 Bezug genommen. Fig. 3 entspricht bis auf die dargestellten Bezugszeichen und Bewegungspfeile der Fig. 1. In der gezeigten Ausführungsform umfasst die bewegliche Verbindung 40 zwischen der Kopfeinheit 4 und dem Haltearm 38 eine Translationskopplung 42 (siehe insbesondere Fig. 3), welche eine lineare Translationsbewegung der Kopfeinheit 4 in Bezug auf den Haltearm 38 bezüglich einer Translationsrichtung T erlaubt, welche zumindest im Wesentlichen parallel zur Sichtrichtung eines von der Stützauflage 6 gestützten Kopfes liegt. Die Translationskopplung 42 umfasst ein kopfeinheitseitiges Kopplungselement 44, an welchem die Kopfeinheit 4 angebracht ist, sowie ein haltearmseitiges Kopplungselement 46, welches an dem Haltearm 38 angebracht, insbesondere fest angebracht, ist. An dem kopfeinheitseitigen Kopplungselement 44 ist ein Bolzen 48 angebracht, welcher das haltearmseitige Kopplungselement 46 bezüglich der Translationsrichtung T gleitend durchdringt. Aufgrund der gleitenden Lagerung des Bolzens 48 in dem haltearmseitigen Kopplungselement 46 lässt sich das kopfeinheitseitige Kopplungselement 44 mit der daran angebrachten Kopfeinheit 4 bezüglich der Translationsrichtung T auf den Haltearm 38 zu oder von ihm weg bewegen. In Richtung auf den Haltearm 38 zu kann ein Eingriff des kopfeinheitseitigen Kopplungselements 44 mit dem haltearmseitigen Kopplungselement 46 einen die Bewegung begrenzenden Anschlag bilden. Eine Bewegung des kopfeinheitseitigen Kopplungselements 44 von dem Haltearm 38 weg ist durch einen an dem Bolzen 48 zum Eingriff mit dem haltearmseitigen Kopplungselement 46 als Verdickung 49 ausgebildeten Anschlag begrenzt. Zur Stabilisierung der Translationskopplung 42 sind in der gezeigten Ausführungsform beidseitig parallel zu dem Bolzen 48 Führungsstangen 51 an dem kopfeinheitseitigen Kopplungselement 44 befestigt und bezüglich der Translationsrichtung T gleitend in dem haltearmseitiges Kopplungselement 46 geführt.

Zwischen den Kopplungselementen 44, 46 der Translationskopplung 42 ist eine Federeinrichtung 50 in Form einer Spiralfeder vorgesehen. Wird das kopfeinheitseitige Kopplungselement 44 auf den Haltearm 38 zubewegt, wird die Federeinrichtung 50 gespannt, so dass eine Rückstellkraft entsteht, welche das kopfeinheitseitige Kopplungselement 44 bezüglich der Translationsrichtung T von dem Haltearm 38 weg beaufschlagt. Lehnt ein Patient seinen Kopf an die Stützauflage 6, wird aufgrund der Auflagekraft des Kopfes auf die Stützauflage 6, insbesondere auf die Nackenstütze 8, die Kopfeinheit 4 über die Translationskopplung 42 entgegen der Federeinrichtung 50 bezüglich der Translationsrichtung T auf den Haltearm 38 zu bewegt. An einem bestimmten Punkt halten sich die von dem Kopf auf die Stützauflage 6 ausgeübte Auflagekraft und die Rückstellkraft der Feder 50 die Waage, so dass ein stationärer Zustand erreicht wird, in dem der Kopf an der Stützauflage 6 gelagert ist. Verringert oder verstärkt sich die Auflagekraft des Kopfes auf die Stützauflage 6, beispielsweise durch eine Bewegung des Kopfes, passt sich die Translationskopplung 42 automatisch an.

Die bewegliche Verbindung 40 zwischen der Kopfeinheit 4 und dem Haltearm 38 umfasst zudem eine erste Drehkopplung 52. Besonders gut erkennbar ist die erste Drehkopplung 52 neben Fig. 3 in der ausschnittsweisen Rückansicht in Fig. 4A. Die erste Drehkopplung 52 erlaubt eine Schwenkbewegung der Kopfeinheit 4 um eine erste Achse A, die bezüglich eines von der Stützauflage 6 gestützten Kopfes zumindest im Wesentlichen parallel zu einer dorso-kaudalen Achse (Oben-Unten-Richtung) verläuft. Eine entsprechende Schwenkbewegung der Kopfeinheit 4 ist in den Fig. 3 und 4A durch den Pfeil V1 dargestellt. In der gezeigten Ausführungsform ist die erste Drehkopplung 52 dadurch realisiert, dass ein an dem kopfeinheitseitigen Kopplungselement 44 der Translationskopplung 42 befestigter Bolzen 54 um die erste Achse A drehbar an der Kopfeinheit 4 angebracht ist. Es wäre auch denkbar, den Bolzen 54 an der Kopfeinheit 4 fest und an dem kopfeinheitseitigen Kopplungselement 44 drehbar anzubringen. Die erste Drehkopplung 52 ermöglicht der Kopfeinheit 4 gegenüber dem feststehenden Haltearm 38 einer Bewegung des Kopfes zu folgen, welche einem Drehen des Kopfes nach links oder rechts (wie bei einem Kopfschütteln) entspricht. Dreht der Patient seinen von der Stützauflage 6 abgestützten Kopf nach links oder rechts, wird über Kontakt mit den Seitenstützen 10, 12 der Stützauflage 6 die Kopfeinheit 4 dazu veranlasst, der Bewegung des Kopfes zu folgen.

Die bewegliche Verbindung 40 zwischen der Kopfeinheit 4 und dem Haltearm 38 umfasst zudem eine zweite Drehkopplung 56. Besonders gut erkennbar ist die zweite Drehkopplung 56 neben Fig. 3 in der ausschnittsweisen Seitenansicht auf die Vorrichtung 1 in Fig. 4B. Die zweite Drehkopplung 56 erlaubt eine Schwenkbewegung der Kopfeinheit 4 in Bezug auf den feststehenden Haltearm 38 um eine zweite Achse B, welche bezüglich eines von der Stützauflage 6 gestützten Kopfes zumindest im Wesentlichen parallel zu einer Ohr-Ohr-Achse (laterale Richtung) verläuft (in Fig. 4B senkrecht zur Zeichenebene). In der gezeigten Ausführungsform ist die zweite Drehkopplung 56 dadurch realisiert, dass ein an dem Bolzen 54 der ersten Drehkopplung 52 angebrachtes Rahmenelement 58 an zwei entlang der zweiten Achse B voneinander beabstandeten Stellen jeweils mit einem Drehbolzen 61 um die zweite Achse B schwenkbar mit einem mit der Kopfeinheit 4 verbundenen Halteelement 60 (siehe auch Fig. 4A und 4C) verbunden ist. Aufgrund der zweiten Drehkopplung 56 kann die Kopfeinheit 4 gegenüber dem feststehenden Haltearm 38 einer Bewegung des Kopfes folgen, welche einem Neigen nach oben oder unten (wie bei einem Nicken) entspricht. Eine entsprechende Drehbewegung der Kopfeinheit 4 ist in den Fig. 3 und 4B durch den Pfeil V2 dargestellt. Führt der Patient eine entsprechende Kopfbewegung aus, ändert sich die Auflagekraft des Kopfes auf die Stützauflage 6, insbesondere auf deren Nackenstütze 8. Diese Kraftänderung wird durch die zweite Drehkopplung 56 in eine entsprechende Bewegung der Kopfeinheit 4 in Bezug auf den stationären Haltearm 38 umgesetzt.

Die bewegliche Verbindung 40 zwischen der Kopfeinheit 4 und dem Haltearm 38 umfasst des Weiteren eine dritte Drehkopplung 62. Besonders gut erkennbar ist die dritte Drehkopplung 62 neben Fig. 3 in der ausschnittsweisen Rückansicht in Fig. 4C, welche bis auf die dargestellten Bezugszeichen und Richtungspfeile der Fig. 4A entspricht. Die dritte Drehkopplung 62 erlaubt eine Schwenkbewegung der Kopfeinheit 4 um eine dritte Achse C, welche bezüglich eines von der Stützauflage 6 gestützten Kopfes zumindest im Wesentlichen parallel zu einer Dorso-rostral-Achse (Nase-Hinterkopf- oder Vorne-Hinten-Richtung) verläuft. In der gezeigten Ausführungsform ist die dritte Drehkopplung 62 dadurch realisiert, dass ein an der Rückseite der der Nackenstütze 8 kreisförmig umlaufender Flansch 63 entlang eines Teils seines Umfangs in einer komplementären, halbkreisförmigen Nut 64 des Halteelements 60 aufgenommen ist. Der Flansch 63 der Nackenstütze 8 ist gleitend in der Nut 64 des Halteelements 60 aufgenommen, so dass das die Nackenstütze 8 in dem Halteelement 60 für eine Drehbewegung um die dritte Achse C gelagert ist. So kann die Kopfeinheit 4 aufgrund der dritten Drehkopplung 62 einer Bewegung des Kopfes folgen, welche einem Kippen des Kopfes zu einer Schulter des Patienten entspricht (sowohl in Richtung auf eine linke als auch in Richtung auf eine rechte Schulter hin). Eine entsprechende Drehbewegung der Kopfeinheit 4 ist in den Fig. 3 und 4C durch den Pfeil V3 dargestellt Führt der Patient eine entsprechende Kopfbewegung aus, wird eine resultierende Änderung der Auflagekraft des Kopfes auf die Stützauflage 6, insbesondere über die Seitenstützen 10, 12 der Stützauflage 6, in eine entsprechende Bewegung der Kopfeinheit 4 umgesetzt.

Die Translationskopplung 42 sowie die erste, zweite und dritte Drehkopplung 52, 56, 62 sind als permanent bewegliche Verbindungen ausgebildet und nicht verriegelbar.

Im Folgenden wird die Einstellung der Position der Magnetspule 2 in Bezug auf den Kopf eines Patienten erläutert.

Zu Beginn der Einstellung soll zunächst die Kopfeinheit 4 gegenüber dem Kopf des Patienten in eine definierte Position gebracht werden, welche bei nachfolgenden Sitzungen reproduziert werden kann. Hierzu wird die Kopfeinheit 4 anhand von Merkmalen des Schädelknochens und/oder des Kopfes des Patienten ausgerichtet. Nachdem der Patient seinen Kopf gegen die Nackenstütze 8 der Stützauflage 6 gelegt hat (ggf. in Grobeinstellung auch schon davor), wird eine allgemeine Position der Kopfeinheit 4 mittels der Verstellmöglichkeiten 41 des Haltearms 38 eingestellt. Gemäß der dargestellten Ausführungsform kann dies mittels eines an der Nackenstütze 8 vorgesehenen Inionsuchers zum Lokalisieren des Inions am Hinterkopf des Patienten erfolgen. Der Inionsucher ist als Tastöffnung 66 (siehe Fig. 1) durch die Nackenstütze 8 ausgebildet. Diese Tastöffnung 66 kann bezüglich des Inions am Hinterkopf des Patienten zentriert werden. Neben der Einstellung einer passenden Höhe für die Kopfeinheit 4 wird die Kopfeinheit 4 damit auch am Kopf des Patienten zentriert. Die Verstellmöglichkeiten 41 des Haltearms 38 können nach erfolgter Einstellung arretiert und der Haltearm 38 damit in seinen raumfest installierten Zustand überführt werden.

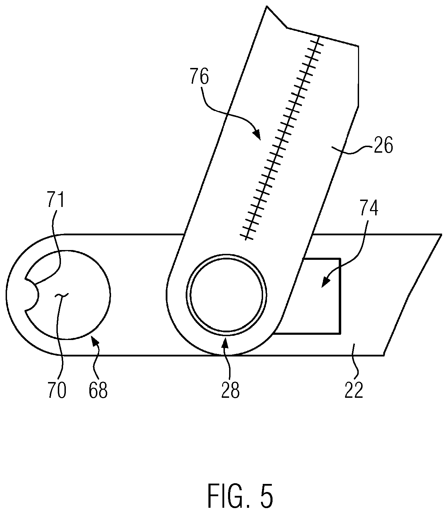

Nachdem die Nackenstütze 8 am Inion ausgerichtet wurde, kann die Kopfeinheit 4 zusätzlich an Ohrmerkmalen (z. B. den Tragi) des Kopfes ausgerichtet werden. Hierzu umfasst die Positioniereinheit 18 an beiden Enden ihres Basisbügels 22 jeweils einen Ohrpositionssucher 68. In dem gezeigten Ausführungsbeispiel umfassen die Ohrpositionssucher 68 jeweils eine Öffnung 70 in dem Basisbügel 22, durch welche sich entsprechende Punkte des Ohrs, wie die Tragi, anvisieren lassen. Die Öffnung 70 kann ein Zielelement, wie einen Vorsprung 71, umfassen, um ein noch genaueres Anvisieren entsprechender Punkte des Ohrs zu ermöglichen. Die Ohrpositionssucher 68 sind beispielsweise in den Fig. 1 und 3 erkennbar. Im Detail ist einer der Ohrpositionssucher 68 in Fig. 5 dargestellt, welche eine vergrößerte Darstellung des Ausschnitts W aus Fig. 2 ist. Zusätzlich oder alternativ können die Ohrpositionssucher 68 jeweils eine Quelle 72 für einen Lichtstrahl, wie beispielsweise eine LED, umfassen, um mit dem Lichtstrahl die Ohrmerkmale anzuvisieren. Zum Ausrichten der Positioniereinheit 18 an den Ohrmerkmalen wird die Positioniereinheit 18 auf der fest an der Stützauflage 6 angebrachten Schiene 24 verschoben, bis die Ohrpositionssucher 68 direkt über den entsprechenden Punkten der Ohren liegen. Dann wird der Basisbügel 22 der Positioniereinheit 18 an der Schiene 24 fixiert. Liegen die Ohrpositionssucher 68 oberhalb oder unterhalb der anvisierten Punkte des Ohres, kann dies durch Lösen des Haltearms 38, entsprechendes Kippen der Kopfeinheit 4 und anschließendes erneutes Fixieren des Haltearms 38 korrigiert werden. Anschließend können die beiden Seitenstützen 10, 12 über die Schwenkverbindungen 14, 16 seitlich an den Kopf herangeführt und dort fixiert werden. Dadurch wird der Kopf in Position relativ zur Kopfeinheit 4 gehalten, um eine gute Kraftübertragung zwischen dem Kopf und der Kopfeinheit 4 zu ermöglichen.

Nachdem die Kopfeinheit 4 als Ganzes in Bezug auf den Kopf ausgerichtet ist, kann mittels eines Verschwenkens des ersten Positionierelements 26 (Pfeil E3) und eines Verschiebens des zweiten Positionierelements 32 entlang des bügelförmigen ersten Positionierelements 26 (Pfeil E4) die Spulenaufnahme 20 in die gewünschte Relativposition zum Kopf des Patienten gebracht werden. Gegebenenfalls kann die Magnetspule 2 in der Spulenhalterung 20 noch in ihrem Abstand auf die Kopfoberfläche (Pfeil E6) und/oder in ihrer Rotationsausrichtung (Pfeil E7) angepasst werden. Auch eine Anpassung der Lage der Spulenaufnahme 20 bezüglich des zweiten Positionierelements 32 gemäß Pfeil E5 ist denkbar. Anschließend werden die Schwenkstellung des bügelförmigen ersten Positionierelements 26 in Bezug auf den Basisbügel 22 sowie die Verschiebestellung des zweiten Positionierelements 32 in Bezug auf das bügelförmige erste Positionierelement 26 und die Lage der Magnetspule 2 in Bezug auf die Spulenaufnahme 20 sowie die Lage der Spulenaufnahme 20 bezüglich des zweiten Positionierelements 32 mit den entsprechenden Feststellelementen festgelegt.

Um für nachfolgende Sitzungen die Einstellung zu vereinfachen, sind Skalen 74, 76 vorgesehen, an denen die Verschwenkstellung des ersten Positionierelements 26 und die Verschiebeposition des zweiten Positionierelements 32 ablesbar sind. Eine erste Skala 74 (Fig. 5) erlaubt das Ablesen der Verschwenkstellung des ersten Positionierelements 26 in Bezug auf den Basisbügel 22. Eine zweite Skala 76 erlaubt ein Ablesen der Verschiebeposition des zweiten Positionierelements 32 entlang des ersten Positionierelements 26. Der Übersichtlichkeit halber sind die Skalen 74, 76 lediglich in den Fig. 1 und 5 schematisch dargestellt. Die erste Skala 74 kann als Winkelskala an dem Basisbügel 22 vorgesehen sein und die zweite Skala 76 kann als Längenskala entlang dem ersten Positionierelement 26 vorgesehen sein. Nachdem eine geeignete Lage der Magnetspule 2 gefunden wurde, können die Skalen 74, 76 abgelesen werden. Zum Ablesen und späteren Wiedereinstellen einer Verschwenkstellungen der Spulenaufnahme 20 in Bezug auf das zweite Positionierelement 32 und einer Drehorientierung der Magnetspule 2 um Bolzen 36 sind Skalen 90, 92 an der Spulenaufnahme 20 und dem Bolzen 36 denkbar. Bei einer nachfolgenden Sitzung können die Verschwenkposition des ersten Positionierelements 26 sowie die Verschiebeposition des zweiten Positionierelements 32 anhand der notierten oder gespeicherten Skalenwerte eingestellt werden und es muss keine erneute experimentelle Bestimmung des optimalen Behandlungspunktes erfolgen. Auch ein Reproduzieren der Verschwenkstellung der Spulenaufnahme 20 in Bezug zu dem zweiten Positionierelement 32 und/oder ein Reproduzieren der Drehorientierung der Magnetspule 2 in Bezug auf die Spulenaufnahme sind anhand der entsprechenden Skalenwerte möglich.

Während die Schiebeverbindung des Basisbügels 22 auf der Schiene 24 (Pfeil E2), die Schwenkverbindung 28, 30 des ersten Positionierelements 26 mit dem Basisbügel 22 (Pfeil E3), die Verschiebeverbindung des zweiten Positionierelements 32 mit dem ersten Positionierelement 26 (Pfeil E4), die Schwenkverbindung der Spulenaufnahme 20 mit dem zweiten Positionierelement 32 (Pfeil E5) und die Drehverbindung der Magnetspule 20 mit der Spulenaufnahme 20 (Pfeil E7) nach ihrer Einstellung fixiert werden, ist die bewegliche Verbindung 40 (Translationskopplung 42 und erste, zweite und dritte Drehkopplung 52, 56, 62) permanent beweglich (Pfeile V1, V2 und V2), insbesondere auch während einer Behandlung des Patienten. Wie oben beschrieben, erlaubt die bewegliche Verbindung 40 mit ihren einzelnen Kopplungen ein Mitführen der Kopfeinheit 4 mit Bewegungen des Kopfes. Bei den resultierenden Bewegungen der Kopfeinheit 4 in Bezug auf den Haltearm 38 bleibt die Lage der Spulenaufnahme 20 relativ zu der Stützauflage 6 und damit die Relativposition der Spule 2 in Bezug auf den Kopf zumindest im Wesentlichen konstant, da die Verschiebeverbindung zwischen der Positioniereinheit 18 und der Schiene 24 arretiert wurde.

## Patentansprüche

1. Vorrichtung (1) zum Positionieren einer Magnetspule (2) zur transkraniellen Magnetstimulation relativ zum Kopf eines Patienten, umfassend:
eine Kopfeinheit (4) mit einer Stützauflage (6) für den Kopf des Patienten und einer mit der Stützauflage (6) verbundenen Positioniereinheit (18) mit einer Spulenaufnahme (20) zum Halten der Magnetspule (2); und
einen die Kopfeinheit (4) haltenden, raumfest installierbaren Haltearm (38),
wobei zwischen der Kopfeinheit (4) und dem Haltearm (38) eine reversibel bewegliche Verbindung (40) derart vorgesehen ist, dass eine Änderung einer durch den Kopf des Patienten auf die Stützauflage (6) ausgeübten Kraft über die reversibel bewegliche (40) eine Relativbewegung zwischen der Kopfeinheit (4) und dem Haltearm (38) bewirkt.

2. Vorrichtung nach Anspruch 1, wobei die reversibel bewegliche Verbindung (40) zwischen der Kopfeinheit (4) und dem Haltearm (38) als permanent bewegliche Verbindung ausgebildet ist.

3. Vorrichtung nach Anspruch 1 oder 2, wobei die Positioniereinheit (18) starr fixierbar mit der Stützauflage (6) verbunden ist.

4. Vorrichtung nach einem der vorangehenden Ansprüche, wobei die Stützauflage (6) derart ausgelegt ist, dass die Änderung der durch den Kopf des Patienten auf die Stützauflage (6) ausgeübten Kraft ein Mitführen der Kopfeinheit (4) mit einer Bewegung des Kopfes des Patienten bewirkt.

5. Vorrichtung nach einem der vorangehenden Ansprüche, wobei die reversibel bewegliche Verbindung (40) zwischen der Kopfeinheit (4) und dem Haltearm (38) dazu ausgelegt ist, der durch den Kopf des Patienten auf die Stützauflage (6) ausgeübten Kraft federnd entgegenzuwirken.

6. Vorrichtung nach einem der vorangehenden Ansprüche, wobei die reversibel bewegliche Verbindung (40) zwischen der Kopfeinheit (4) und dem Haltearm (38) eine Translationskopplung (42) umfasst, welche eine lineare Translationsbewegung der Kopfeinheit (4) in Bezug auf den Haltearm (38) bezüglich einer Translationsrichtung (T), welche vorzugsweise parallel zur Sichtrichtung eines von der Stützauflage (6) gestützten Kopfes liegt, erlaubt, wobei die Translationskopplung (42) vorzugsweise eine Federeinrichtung (50) umfasst, welche dazu ausgelegt ist, bei einer Vergrößerung einer zu der Translationsrichtung (T) parallelen Komponente der durch den Kopf des Patienten auf die Stützauflage (6) ausgeübten Kraft und einer damit einhergehenden linearen Translationsbewegung der Kopfeinheit (4) bezüglich der Translationsrichtung (T) gespannt zu werden, und bei einer nachfolgenden Verringerung der Kraftkomponente die Kopfeinheit (4) zu einer entgegengesetzten linearen Translationsbewegung zu beaufschlagen.

7. Vorrichtung nach einem der vorangehenden Ansprüche, wobei die reversibel bewegliche Verbindung (40) eine erste Drehkopplung (52) umfasst, die eine Schwenkbewegung der Kopfeinheit (4) um eine erste Achse (A) erlaubt, und/oder eine zweite Drehkopplung (56) umfasst, die eine Schwenkbewegung der Kopfeinheit (4) um eine zweite Achse (B) erlaubt, und/oder eine dritte Drehkopplung (62) umfasst, die eine Schwenkbewegung der Kopfeinheit (4) um eine dritte Achse (C) erlaubt, wobei die erste, die zweite und die dritte Achse (A, B, C) vorzugsweise jeweils rechtwinklig zueinander stehen.

8. Vorrichtung nach einem der vorangehenden Ansprüche, wobei die Positioniereinheit (18) zumindest ein Positionierelement (26, 32) umfasst, welches zum Einstellen der Lage der Spulenaufnahme (20) relativ zu der Stützauflage (6) verstellbar ist.

9. Vorrichtung nach einem der vorangehenden Ansprüche, wobei die Positioniereinheit (18) zum Einstellen einer Spulenposition ein verschwenkbares erstes Positionierelement (26) und ein verschiebbar daran angebrachtes zweites Positionierelement (32) umfasst, wobei die Spulenaufnahme (20) fest oder verstellbar an dem zweiten Positionierelement (32) angebracht ist, wobei vorzugsweise zudem zumindest eine Skala (74, 76) vorgesehen ist, an der eine Verschwenkstellung des ersten Positionierelements (26) und/oder eine Verschiebeposition des zweiten Positionierelements (32) ablesbar sind.

10. Vorrichtung nach einem der vorangehenden Ansprüche, wobei die Stützauflage (6) eine Nackenstütze (8) umfasst, welche vorzugsweise gepolstert ist, wobei die Stützauflage (6) vorzugsweise zudem zu beiden Seiten der Nackenstütze (8) jeweils eine Seitenstütze (10, 12) umfasst, wobei die Seitenstützen (10, 12) jeweils derart schwenkbar an der Nackenstütze (8) angebracht sind, dass sie seitlich an einen von der Nackenstütze (8) gestützten Kopf eines Patienten angelegt werden können und in einer Anlageposition in Bezug auf die Nackenstütze (8) verriegelbar sind.

11. Vorrichtung nach Anspruch 10, wobei die Nackenstütze (8) eine Tastöffnung (66) als Inionsucher zum Lokalisieren des Inions eines von der Nackenstütze (8) gestützten Kopfes umfasst.

12. Vorrichtung nach einem der vorangehenden Ansprüche, wobei an der Positioniereinheit (18) eine oder mehrere Ohrpositionssucher (68) vorgesehen sind, mit denen sich markante Ohrpositionen an einem von der Nackenstütze (8) gestützten Kopf anvisieren lassen, wobei die Ohrpositionssucher (68) jeweils eine Öffnung (70) in der Positioniereinheit (18) und/oder eine Quelle (72) für einen Lichtstrahl umfassen.

13. Verfahren zum Positionieren einer Magnetspule (2) relativ zum Kopf eines Patienten, umfassend:
Vorsehen eines raumfest installierten Haltearms (38);
Anbringen einer Kopfeinheit (4) mit einer Stützauflage (6) für den Kopf des Patienten und mit einer mit der Stützauflage (6) verbundenen Positioniereinheit (18), welche eine Spulenaufnahme (20) zum Halten der Magnetspule (2) umfasst, an dem Haltearm (38), so dass der Haltearm (38) die Kopfeinheit (4) hält; und
Einstellen der Positioniereinheit (18) zum Einstellen der Lage der Spulenaufnahme (20) relativ zu der Stützauflage (6) in eine gewünschte Behandlungsposition für die Magnetspule (2),
wobei die Kopfeinheit (4) derart reversibel beweglich an dem Haltearm (38) angebracht wird, dass eine Änderung einer durch den Kopf des Patienten auf die Stützauflage (6) ausgeübten Kraft über eine reversibel bewegliche Verbindung (40) zwischen der Kopfeinheit (4) und dem Haltearm (38) eine Relativbewegung zwischen der Kopfeinheit (4) und dem Haltearm (38) bewirkt.

14. Verfahren nach Anspruch 13, zudem umfassend:
automatisches Mitführen der Kopfeinheit (4) mit einer Bewegung des Kopfes des Patienten bei einer Änderung der durch den Kopf des Patienten auf die Stützauflage (6) ausgeübten Kraft aufgrund der reversibel beweglichen Verbindung (40) zwischen der Kopfeinheit (4) und dem Haltearm (38),
wobei vorzugsweise beim automatischen Mitführen der Kopfeinheit (4) mit der Bewegung des Kopfes des Patienten die Einstellung der Positioniereinheit (18) beibehalten wird und somit die Lage der Spulenaufnahme (20) relativ zu der Stützauflage (6) gleich bleibt.

15. Verfahren nach Anspruch 13 oder 14, zudem umfassend:
federndes Entgegenwirken gegen die durch den Kopf des Patienten auf die Stützauflage (6) ausgeübte Kraft durch die reversibel bewegliche Verbindung (40) zwischen der Kopfeinheit (4) und dem Haltearm (38).

## Claims

1. Device (1) for positioning a magnetic coil (2) for transcranial magnetic stimulation in relation to the head of a patient, comprising:
a head unit (4) with a support (6) for the head of the patient and a positioning unit (18), which is connected to the support (6), with a coil seat (20) for holding the magnetic coil (2); and
a holding arm (38) that holds the head unit (4) and that is configured to be installed firmly,
wherein a reversibly movable connection (40) is provided between the head unit (4) and the holding arm (38) in a way that a change of a force, which is applied onto the support (6) by the head of the patient, leads to a relative movement between the head unit (4) and the holding arm (38) through the reversibly movable connection (40).

2. Device according to Claim 1, wherein the reversibly movable connection (40) between the head unit (4) and the holding arm (38) is formed as a permanently movable connection.

3. Device according to Claim 1 or 2, wherein the positioning unit (18) is connected to the support (6) in a way that it can be fixed rigidly.

4. Device according to one of the preceding claims, wherein the support (6) is formed in such a way that the change of the force, which is applied onto the support (6) by the head of the patient, leads to the head unit (4) being guided along with a movement of the patient's head.

5. Device according to one of the preceding claims, wherein the reversibly movable connection (40) between the head unit (4) and the holding arm (38) is configured to counteract the force, which is applied onto the support (6) by the patient's head, in an elastic way.

6. Device according to one of the preceding claims, wherein the reversibly movable connection (40) between the head unit (4) and the holding arm (38) comprises a translation coupling (42) that allows for a linear translation movement of the head unit (4) in relation to the holding arm (38) with respect to a translation direction (T) that is preferably parallel to the viewing direction of a head supported by the support (6), wherein the translation coupling (42) preferably comprises a spring device (50) that is configured to be tensioned in case of an increase of a component of the force applied onto the support (6) by the patient's head that is parallel to the translation direction (T) and a linear translation movement of the head unit (4) with respect to the transport direction that is entailed by said increase, and to act upon the head unit (4) in case of a subsequent reduction of the force component towards an opposed linear translation movement.

7. Device according to one of the preceding claims, wherein the reversibly movable connection (40) comprises a first rotary coupling (52) that allows for a swivel movement of the head unit (4) about a first axis (A) and/or comprises a second rotary coupling (56) that allows for a swivel movement of the head unit (4) about a second axis (B), and/or comprises a third rotary coupling (62) that allows for a swivel movement of the head unit (4) about a third axis (C), wherein the first, the second and the third axis (A, B, C) are each positioned at least essentially at a right angle in relation to one another.

8. Device according to one of the preceding claims, wherein the positioning unit (18) comprises at least one positioning element (26, 32) that can be adjusted for adjusting the position of the coil seat (20) in relation to the support (6).

9. Device according to one of the preceding claims, wherein for adjusting a coil position, the positioning unit (18) comprises a tiltable first positioning element (26) and a second positioning element (32) that is installed on said first positioning element (26) in a shiftable way, wherein the coil seat (20) is installed firmly or adjustably at the second positioning element (32), wherein preferably further at least one scale (74, 76) is provided from which a swivel position of the first positioning element (26) and/or a shifting position of the second positioning element (32) can be read.

10. Device according to one of the preceding claims, wherein the support (6) comprises a neckrest (8) that is in particular cushioned,wherein preferably the support (6) further comprises respectively one side support (10, 12) to both sides of the neckrest (8), wherein the side supports (10, 12) are each installed tiltably on the neckrest (8) in such a way that they can be fitted laterally to a patient's head that is supported by the neckrest (8) and that they can be locked in a placement position in relation to the neckrest (8).

11. Device according to Claim 10, wherein the neckrest (8) comprises a sensorial opening (66) as an inion searcher for localizing the inion of a head that is supported by the neckrest (8).

12. Device according to one of the preceding claims, wherein one or multiple ear position searchers (68) are provided on the positioning unit (18) by means of which distinctive ear positions on a head that is supported by the neckrest (8) can be targeted, wherein the ear position searchers (68) respectively comprise an opening (70) in the positioning unit (18) and/or a source (72) for a light beam.

13. Method for positioning of a magnetic coil (2) in relation to the head of a patient, comprising:
providing a firmly installed holding arm (38);
installing a head unit (4) with a support (6) for the head of the patient and with a positioning unit (18) that is connected to the support (6) and that comprises a coil seat (20) for holding the magnetic coil (2) on the holding arm (38) so that the holding arm (38) holds the head unit (4); and
adjusting the positioning unit (18) to adjust the position of the coil seat (20) in relation to the support (6) to a desired treatment position for the magnetic coil (2),
wherein the head unit (4) is installed on the holding arm (38) in such a reversibly movable way that a change of a force, which is applied onto the support (6) by the patient's head, leads to a relative movement between the head unit (4) and the holding arm (38) through the reversibly movable connection (40) between the head unit (4) and the holding arm (38).

14. Method according to Claim 13, further comprising:
automated guiding of the head unit (4) along with a movement of the patient's head in case of a change of the force, which is applied onto the support (6) by the patient's head, due to the reversibly movable connection (40) between the head unit (4) and the holding arm (38),wherein preferably during automated guiding of the head unit (4) along with the movement of the patient's head, the setting of the positioning unit (18) is maintained and therefore the position of the coil seat (20) in relation to the support (6) remains the same.

15. Method according to Claim 13 or 14, further comprising:
elastic counteracting against the force, which is applied onto the support (6) by the patient's head, by the movable connection (40) between the head unit (4) and the holding arm (38).

## Revendications

1. Dispositif (1) de positionnement d'une bobine magnétique (2) de stimulation magnétique transcrânienne par rapport à la tête d'un patient, comprenant :
une unité tête (4) pourvue d'un support d'appui (6) pour la tête du patient et d'une unité de positionnement (18) reliée au support d'appui (6) avec un support de bobine (20) permettant le maintien de la bobine magnétique (2) ; et
un bras de maintien (38) pouvant être installé de manière fixe dans la pièce pour maintenir l'unité tête (4) entre l'unité tête (4) et le bras de maintien (38), une liaison mobile réversible (40) étant prévue de manière à ce que le changement d'une force exercée sur le support d'appui (6) par la tête du patient entraîne, par l'intermédiaire de la liaison mobile réversible (40), un mouvement relatif entre l'unité tête (4) et le bras de maintien (38).

2. Dispositif selon la revendication 1, la liaison mobile réversible (40) entre l'unité tête (4) et le bras de maintien (38) étant conçue en tant que liaison mobile permanente.

3. Dispositif selon la revendication 1 ou 2, l'unité de positionnement (18) étant reliée au support d'appui (6) de manière fixe et rigide.

4. Dispositif selon l'une des revendications précédentes, le support d'appui (6) étant conçu de telle manière que le changement de la force exercée par la tête du patient sur le support d'appui (6) amène l'unité tête (4) à se déplacer avec chaque mouvement de tête du patient.

5. Dispositif selon l'une des revendications précédentes, la liaison mobile réversible (40) entre l'unité tête (4) et le bras de maintien (38) étant conçue pour agir en contre-réaction élastique vis-à-vis de la force exercée par la tête du patient sur le support d'appui (6).

6. Dispositif selon l'une des revendications précédentes, la liaison mobile réversible (40) comprenant, entre l'unité tête (4) et le bras de maintien (38), un accouplement de translation (42) qui permet un mouvement de translation linéaire de l'unité tête (4) par rapport au bras de maintien (38), et ce dans un sens de translation (T), qui est de préférence parallèle au sens du regard d'une tête soutenue par le support d'appui (6), l'accouplement de translation (42) comprenant de préférence un dispositif de ressort (50) qui est conçu pour être tendu par rapport au sens de la translation (T) dans le cas d'une augmentation d'une composante - parallèle au sens de la translation (T) - de la force exercée par la tête du patient sur le support d'appui (6) s'accompagnant d'un mouvement de translation linéaire de l'unité tête (4), et pour contraindre l'unité tête (4) à un mouvement de translation linéaire opposé dans le cas d'une diminution subséquente de la composante de force.

7. Dispositif selon l'une des revendications précédentes, la liaison mobile réversible (40) comprenant un premier accouplement rotatif (52) qui permet un mouvement de pivotement de l'unité tête (4) autour d'un premier axe (A), et/ou un second accouplement rotatif (56) qui permet un mouvement de pivotement de l'unité tête (4) autour d'un second axe (B), et/ou un troisième accouplement rotatif (62) qui permet un mouvement de pivotement de l'unité tête (4) autour d'un troisième axe (C), le premier, le second et le troisième axe (A, B, C) se situant préférablement sous des angles droits les uns par rapport aux autres.

8. Dispositif selon l'une des revendications précédentes, l'unité de positionnement (18) comprenant au moins un élément de positionnement (26, 32) qui est déplaçable par rapport au support d'appui (6) de façon à permettre le réglage de la position du support de bobine (20).

9. Dispositif selon l'une des revendications précédentes, l'unité de positionnement (18) comprenant - pour le réglage d'une position de bobine - un premier élément de positionnement pivotant (26) et un second élément de positionnement (32) fixé de façon déplaçable sur le premier élément de positionnement (26), le support de bobine (20) étant installé de manière fixe ou déplaçable sur le second élément de positionnement (32) ; en outre, il est prévu de préférence au moins une échelle graduée (74, 76), sur laquelle peuvent être lues une position de pivotement du premier élément de positionnement (26) et/ou une position de déplacement du second élément de positionnement (32).

10. Dispositif selon l'une des revendications précédentes, le support d'appui (6) comprenant un appuie-tête (8) rembourré de préférence, le support d'appui (6) comprenant de préférence aussi - des deux côtés de l'appuie-tête (8) - respectivement un support latéral (10, 12) ; les supports latéraux (10, 12) étant fixés chacun de telle manière pivotante sur l'appuie-tête (8) qu'ils peuvent être placés latéralement contre la tête d'un patient supportée par l'appuie-tête (8) et qu'ils peuvent être verrouillés par rapport à l'appuie-tête (8) lorsqu'ils se trouvent dans une position d'appui.

11. Dispositif selon la revendication 10, l'appuie-tête (8) comprenant une ouverture de détection (66) qui fonctionne comme un détecteur d'inion pour la localisation de l'inion d'une tête supportée par l'appuie-tête (8).

12. Dispositif selon l'une des revendications précédentes, un ou plusieurs détecteurs de position d'oreille (68) étant prévus sur l'unité de positionnement (18), à l'aide desquels des positions d'oreille notables peuvent être visées au niveau d'une tête supportée par l'appuie-tête (8), les détecteurs de position d'oreille (68) comprenant chacun une ouverture (70) dans l'unité de positionnement (18) et/ou une source (72) pour un faisceau de lumière.

13. Procédé de positionnement d'une bobine magnétique (2) par rapport à la tête d'un patient, comprenant :
la fourniture d'un bras de maintien (38) installé de manière fixe dans la pièce ;
la fixation sur le bras de maintien (38) d'une unité tête (4) avec un support d'appui (6) pour la tête du patient et avec une unité de positionnement (18) - reliée à un support d'appui (6) - qui comprend un support de bobine (20) permettant de maintenir la bobine magnétique (2), de sorte que le bras de maintien (38) maintient l'unité tête (4) ; et
l'ajustement de l'unité de positionnement (18) pour le réglage de la position du support de bobine (20) par rapport au support d'appui (6) jusqu'à obtenir une position de traitement souhaitée pour la bobine magnétique (2),
l'unité tête (4) étant fixée sur le bras de maintien (38) d'une manière mobile réversible telle qu'un changement d'une force exercée par la tête du patient sur le support d'appui (6) entraîne, par l'intermédiaire d'une liaison mobile réversible (40) entre l'unité tête (4) et le bras de maintien (38), un mouvement relatif entre l'unité tête (4) et le bras de maintien (38).

14. Procédé selon la revendication 13, comprenant en outre :
l'entraînement automatique de l'unité tête (4) par un mouvement de la tête du patient, dans l'éventualité d'un changement d'une force exercée sur le support d'appui (6) par la tête du patient, du fait de la liaison mobile réversible (40) entre l'unité tête (4) et le bras de maintien (38),
l'ajustement de l'unité de positionnement (18) étant de préférence conservé lors de l'entraînement automatique de l'unité tête (4) par le mouvement de la tête du patient, et la position du support de bobine (20) demeurant ainsi identique par rapport au support d'appui (6).

15. Procédé selon la revendication 13 ou 14, comprenant en outre :
une force d'opposition élastique permettant de contrecarrer la force exercée par la tête du patient sur le support d'appui (6), du fait de la liaison mobile réversible (40) entre l'unité tête (4) et le bras de maintien (38).
